# EUROPEAN PATENT SPECIFICATION

(11) **EP 1 293 210 B1**
(45) Date of publication and mention of the grant of the patent: **19.11.2003**
(21) Application number: 01201945.1
(22) Date of filing: 23.05.2001
(51) Int. Cl.: A61K 31/57, A61P 15/18

(54) **Means and method for hormonal contraception**
Zubereitungen für die hormonale Kontrazeption
Kit et méthode contraceptive hormonale

(43) Date of publication of application: 19.03.2003
(73) Proprietor: Pantarhei Bioscience B.V., 3701 CH Zeist (NL)
(72) Inventor: Van Beek, Agatha Antonia Magdalena, NL-5403 Am Uden (NL); Coelingh Bennink, Herman Jan Tijmen, NL-3971 Be Driebergen (NL)
(74) Representative: van Westenbrugge, Andries

(56) References cited:
- DE-A- 4 224 534
- DE-C- 4 429 374
- US-A- 4 921 843
- US-A- 5 662 927

## Description

### TECHNICAL FIELD

The present invention is concerned with a kit containing a plurality of hormone units for use in a contraceptive method which consists of two alternating consecutive phases, sometimes referred to as a sequential method or sequential regimen. This method comprises administering to a female of childbearing capability during one phase one or more hormone units containing estrogen in a therapeutically effective amount to inhibit ovulation and during the other phase one or more hormone units, containing a combination of estrogen and progestogen in a therapeutically effective amount to inhibit ovulation and to transform the endometrium from a proliferative into a secretory state. The method according to the invention, unlike virtually all methods used to date, does not include an interval of about 2-7 days during which a placebo or no daily units are administered.

### BACKGROUND OF THE INVENTION

Kits for use in a sequential contraceptive method as described above are known in the art. EP-A 0 628 312 (Jenapharm) describes a method comprising one or more phases wherein one phase uses a combination of biogenic estrogen, synthetic estrogen and progestogen and the other phases may use a placebo, or a synthetic or biogenic progestogen, or a synthetic or biogenic estrogen, or a combination of biogenic estrogen, synthetic estrogen and progestogen, or a combination of synthetic estrogen and progestogen. An examples is given of a regimen which consists of 2 phases, one phase of 21 days using the combination of biogenic estrogen, synthetic estrogen and progestogen and another phase of 7 days using only a biogenic estrogen or no hormone at all.

DE-A 42 24 534 (Ehrlich et al.) is concerned with a sequential contraceptive method that consists of one phase of 5-14 days during which an estrogen preparation is administered in an therapeutically effective amount to cause disturbance of the follicle stimulation and another phase of 14-23 days during which a combination of estrogen and progestogen preparation is administered in a therapeutically effective amount to inhibit ovulation, and wherein the ethinyl estradiol concentration in the estrogen preparation, if used, is below 30µg. In all the examples the same estrogen is used in both phases.

WO 95/17895 (Ehrlich et al.) describes a sequential method of hormonal contraception comprising the steps of administering a first hormonal component composed of a plurality of daily hormone units including a therapeutically effective amount of an estrogen preparation to cause disturbance of the follicle stimulation, and a second hormonal component composed of a plurality of daily hormone units including a therapeutically effective amount of an estrogen preparation and a progestogen preparation to inhibit ovulation, providing the daily units of the first hormonal component in a plurality which is lower than the plurality of daily units of the second hormonal component, and wherein the second hormonal component is not a combination of a biologically produced estrogen and a synthetic estrogen. In all the examples the same estrogen is used in both phases.

US 4,921,843 is concerned with a method of contraception which comprises administering to a human female daily during the 7 days following menses a first composition containing as sole contraceptively active ingredient an estrogenic compound and thereafter through day 28 of her menstrual cycle at least one follow-up composition containing a contraceptively effective daily dosage of progestin and optionally an estrogen. A number of estrogens are mentioned, including both synthetic estrogens and a biogenic estrogen. The examples illustrate the use of only synthetic estrogen (ethinyl estradiol) in both compositions.

DE-C 44 29 374 relates to a contraceptive method which consists of 3 different phases, wherein each phase comprises the administration of a biogenic estrogen. The use of synthetic estrogens is clearly advised against. All the examples illustrate the exclusive use of biogenic estrogens.

### SUMMARY OF THE INVENTION

Currently on the market there are a number of contraceptive preparations which can be classified into two general types. The first one are known as monophasic preparations. These contain a constant amount of estrogen and progestogen. Undesired side effects with these pills depend on the balance between the estrogen and progestogen component of the pill. For example, with a relatively dominant progestogen pill, the preparation will, over time, result in a depletion of both estrogen and progesterone receptors. The result which can be expected is an understimulated or atrophic endometrium which may eventually cause either on-pill amenorrhea or breakthrough bleeding or spotting due to poor epithelialisation. On the other hand, with a relatively dominant estrogenic preparation, it is possible that prolonged use will result in endometrial growth with the development of unsupported fragile stroma and subsequent spotting or breakthrough bleeding.

Newer preparations known as triphasic preparations have varying levels of estrogen and progestogen; in most cases consisting of relatively constant levels of estrogen with a step-wise increase in progestogen throughout the cycle. This pattern of estrogen and progestogen administration results in a relatively dominant estrogenic formulation at the beginning of the package with increasing progestogenic activity toward the end of the package. Endometrial stability is believed to be better with these pills since the estrogenic activity at the beginning of the package induces both estrogen and progesterone receptors making the endometrium sensitive to the increased levels of progestogen towards the end of the package. The progestogenic activity produces denser, more stable endometrial stroma although the relatively long duration of progestogenic exposure, toward the end of the package, may still lead to decreased estrogen and progesterone receptors and activity. A significant problem with this type of preparation is the low dose of hormones at the beginning of the package which makes these pills vulnerable to drug interactions or missed pills which may lead to escape ovulation. The beginning of the package is the critical time in terms of escape ovulation since the user has just completed a 7 day drug-free interval during which follicular development may begin.

Almost all of the methods of hormonal contraception currently on the market have in common that they are based on a regimen which involves an administration-free interval of about 7 days whereby withdrawal bleeding simulating the natural menses occurs. Thus 21 day intervals of hormone administration alternate with 7 days during which no hormones are administered.

Another characteristic of these methods is the combined use of estrogen and progestogen throughout the administration regimen. So called "unopposed" administration of estrogen has been associated with endometrial proliferation in menopausal women who received estrogen replacement therapy. It is widely accepted that continuous "unopposed" estrogen therapy substantially increases the risk of endometrial cancer. In order to counteract the negative effects of unopposed estrogen therapy, adjunctive progestogen treatment is nowadays commonly applied, also in the field of hormonal contraception. Regular progestogen administration is believed to inhibit the continual estrogen stimulation of the endometrium through an anti-proliferative effect and appears to reduce the incidence of endometrial carcinoma in post-menopausal women receiving estrogen replacement therapy [Beral V., Banks E., Reeves G., Appleby P., "Use of HRT and the subsequent risk of cancer", J. Epidemiol. Biostat. (1999), 4(3), 191-210].

The three aspects that are considered to be most important in hormonal contraception are contraceptive reliability, cycle control and minimum side-effects. It is a commonly held belief that the contraceptive reliability is critically dependent on the inhibition of ovulation by the progestogen constituent. Added thereto are the peripheral effects of the progestogen on the cervix, fallopian tubes, and endometrium. In combined ethinyl estradiol/progestogen preparations the daily progestogen dose is always significantly higher compared to the ovulation inhibiting dose of the progestogen alone. This has two major reasons. Firstly the addition of ethinyl estradiol increases the level of Sex Hormone Binding Globulin (SHBG). SHBG binds and inactivates both estrogens and progestogens. The free, non SHBG bound fraction of those steroids is biologically active. Due to this mechanism a higher progestogen dose is needed in combined preparations to achieve a sufficiently high free progestogen level. Secondly when adding an estrogen to the contraceptive regimen more progestogen is needed to counteract estrogen induced endometrial proliferation (monophasics/continuous combined pills) or transform the endometrium from proliferation to secretion (sequential pills). Although estrogen itself is commonly held to be added to contraceptive regimens to achieve acceptable vaginal bleeding pattern, estrogens also inhibit ovulation in a dose dependent way. Therefore an amount of estrogen that is sufficiently biologically active, either a biogenic or a synthetic estrogen or a combination thereof, will inhibit ovulation. However biogenic estrogens alone require such high dosages for inhibition of ovulation that side-effects prevent the use of such compounds. Combined ethinyl estradiol progestogen preparations that are taken over three weeks followed by an administration pause of 6-7 days have, hitherto, shown the greatest contraceptive reliability. Thus it is not surprising that these combined preparations have gained immense popularity.

While the combination preparations described above offer the greatest contraceptive reliability among known ovulation inhibitors, the best cycle control (i.e. regular withdrawal menses (expected bleedings) with optimally few intermenses (unexpected bleedings)) is achieved with the use of sequential preparations, including those of the type that affect proliferation of the endometrium. This is due to the (usually) 7-day action of the estrogen (unimpeded by progestogen) prior to the progestogen being added. Beginning with the eighth day, further proliferation is inhibited and the endometrium is thereby altered. A menstruation-like withdrawal bleeding occurs approximately 2 to 3 days after the last estrogen-progestogen dose is administered. In contrast, the proliferation of the endometrium is reduced from the very outset with the use of combination preparations, so that the cycle control in the latter case is poorer than with use of the sequential preparations.

An essential element of the sequential method according to the invention is the application in one phase of a synthetic estrogen in the absence of a progestogen, and the combined application of a biogenic estrogen and a progestogen in the other phase. Thus the method according to the invention consists of two alternating consecutive phases, wherein during one phase a therapeutically effective amount of synthetic estrogen or a combination of synthetic and biogenic estrogen is administered to inhibit ovolation and during the other phase a combination of biogenic estrogen and progestogen is administered in a therapeutically effective amount to inhibit ovulation and to transform the endometrium from a proliferative into a secretory state.

The hormone regimen used in the present method is very compatible with natural female physiology. The menstruation cycle of females effectively consists of 2 hormonal phases, a proliferation phase regulated by estrogen and a secretion phase which is regulated by the combination of estrogen and progesterone. The present method uses a very similar pattern which offers the advantage that, for instance, estrogen withdrawal symptoms, common to most commercially available contraception regimens, do not occur.

The present invention relates to a sequential method that exhibits an optimum combination of contraceptive reliability, cycle control and minimum side-effects. In terms of contraceptive reliability and side-effects the sequential method of the invention performs better than methods that make use of combined preparations. Due to the fact that the present method docs not make use of administration-free intervals, the risk that mistakes in the administration will lead to escape ovulations is much lower than in methods using combined preparations and an administration-free interval. The combination of a pause of 6-7 days during which significant follicular development occurs and the well documented bad compliance of many pill-users (30%-40% forget pills occasionally) cause an increased risk of escape ovulation "around" the pill pause. This results in "real life" pregnancy rates of 3-8% per year. By removing the pause and administering ovulation inhibiting steroids daily, the risk of escape ovulation is much lower with the proposed newly invented regimen. As regards side-effects the present method offers the advantage that it employs a biogenic estrogen during the second part of the cycle wherein adequate reliability can be achieved without the use of a synthetic estrogen like ethinyl estradiol. Because biogenic estrogens are naturally present in the female body, side-effects do not normally occur as long as serum levels do not substantially exceed naturally occurring concentrations. With synthetic estrogens there is a (dose dependent) risk of undesirable side-effects, such as thromboembolism, fluid retention and breast pain. Also side-effects that occur as a result of chronic fluctuations in blood serum estrogen levels, e.g. estrogen withdrawal symptoms, are avoided by the present method.

In terms of cycle control the present sequential method also clearly outperforms the known methods. In particular the present method offers a much more predictable bleeding pattern as well as better metabolic safety (less of a burden to the liver due to (a) the relatively low dosage of synthetic estrogen and (b) the use of a biogenic estrogen during part of the regimen) in combination with an improved feeling of well-being that is based on the continuous administration of estrogen throughout the cycle.

In comparison to the known sequential methods such as those disclosed in EP-A 0 628 312 (Jenapharm), DE-A 42 24 534 (Ehrlich et al.) and WO 95/17895 (Ehrlich et al.), the present method offers the advantage that it provides maximum reliability by employing a synthetic estrogen during the estrogenic phase while minimising the disadvantageous side-effects of synthetic estrogen by employing biogenic estrogen during the progestogenic phase, which phase usually represents a significant part of the cycle. The use of only biogenic estrogen during the estrogenic phase is insufficient to adequately inhibit follicular development, thereby increasing the risk of ovulation.

### DETAILED DESCRIPTION OF THE INVENTION

One embodiment of the present invention is concerned with a kit containing a plurality of daily hormone units for use in a contraceptive method, the plurality of daily hormone units consisting of:
a) one or more daily hormone units containing synthetic estrogen or a combination of synthetic estrogen and biogenic estrogen in an amount equivalent to 3-40 µg ethinyl estradiol and
b) at least 10 daily hormone units containing biogenic estrogen in an amount equivalent to 0.5-5 mg 17beta-estradiol and progestogen in an amount equivalent to 30-750 µg levonorgestrel.

The one or more daily units containing synthetic estrogen are for use during the estrogenic phase of the method described below, whereas the at least 10 units containing the combination of biogenic estrogen and progestogen are for use during the progestogenic phase. The preferred. embodiments described below in relation to the contraceptive method of the invention are equally valid for the above kit, unless they relate to parameters which are only meaningful in relation to a method, i.e. procedure of contraception.

Another embodiment of the invention relates to a kit containing hormone units for use in a contraceptive method that consists of two alternating consecutive phases - an estrogenic and a progestogenic phase - said method comprising administering to a female of childbearing capability
a) during the estrogenic phase one or more hormone units to provide a therapeutically effective amount of synthetic estrogen or a combination of synthetic estrogen and biogenic estrogen to inhibit ovulation and
b) during the progestogenic phase one or more hormone units to provide a combination of biogenic estrogen and progestogen in a therapeutically effective amount to inhibit ovulation and to transform the endometrium from a proliferative into a secretory state,
wherein the progestogenic phase encompasses a period of at least 10 days and the two consecutive phases together encompass a period of 20-35 days.

The term female, whenever referred to in here, relates to female mammals. Preferably the female mammal is a homo sapiens. For homo sapiens females are usually biologically capable of child bearing between the age of 12 and 55.

The hormone units according to the invention may be administered orally, parenterally, sublingually, transdermally, intravaginally, intranasally or buccally. The daily hormonal units can suitably be administered orally, transdermally or intravaginally. Methods for transdermal administration including the associated methods for manufacturing such systems are well known in the art. In this connection, reference may be had to U.S. Pat. Nos. 4,752,478, 4,685,911, 4,438,139 and 4,291,014.

An important element of the contraceptive method according to the invention is that it involves no major administration-free intervals. The present method allows administration-free intervals of up to 2 days without a serious decrease in reliability. Hence the method encompasses a regimen which includes one or more administration-free intervals of up to 2 days. In a more preferred embodiment, the contraceptive method comprises the uninterrupted daily administration of a hormone unit from the plurality of daily hormone units during the cycle between 2 menses, more preferably during at least 3 of such cycles.

For most human females the natural interval between menses is somewhere between 20 and 35 days. To mimic the natural cyclic menses pattern, it is preferred that the plurality of hormone units consists of 20 to 35 daily hormone units. Most preferably the plurality of daily hormone units consists of 28 daily hormone units.

In accordance with the invention, during the estrogenic phase, one or more hormone units are administered to provide a therapeutically effective amount of synthetic estrogen or a combination of synthetic and biogenic estrogen to inhibit ovulation. During said estrogenic phase it was found to be advantageous to administer a combination of synthetic and biogenic estrogen as this enables a further reduction of the dose of synthetic estrogen needed to achieve ovulation inhibition. In addition these units preferably do not contain progestogen as the presence of such hormone may adversely affect the bleeding pattern. Most preferably the units containing the synthetic estrogen, as used during the estrogen phase, are free of progestogen, anti-progestogen and androgen.

In the present method, during the progestogenic phase, hormone units are administered to provide a combination of biogenic estrogen and progestrogen in a therapeutically effective amount to inhibit ovulation and to transform the endometrium from a proliferative into a secretory state. As explained above, it is advantageous not to use synthetic estrogen during this phase. Hence in a preferred embodiment of the invention the units containing a combination of biogenic estrogen and progestogen do not contain a synthetic estrogen.

The administration of estrogen as the only hormonally active ingredient during the estrogenic phase does not interfere with the withdrawal bleeding which occurs after the progestogenic phase due to discontinuation (withdrawal) of the progestogen administration. Unopposed estrogen administration causes stimulation of the progesterone receptors in the endometrium, allowing progestogens to be optimally effective in transforming the endometrium in a successive phase. As a result of this, a reduced rate of intermenstrual breakthrough bleeding, compared to conventionally combined low-dose preparations, is achieved. It is preferred to employ synthetic, or a combination of synthetic and biogenic estrogen during the estrogenic phase so as to ensure an optimum level of contraceptive reliability.

In a preferred embodiment of the invention the daily hormone units for use during the estrogenic phase contain the synthetic estrogen, or the combination of synthetic and biogenic estrogen, in an amount equivalent to 3-40 µg ethinyl estradiol. It is an important aspect of the present invention that it enables the use of synthetic estrogen at very reduced levels without a significant reduction in contraceptive reliability. Whereas most commercially available kits comprise daily hormone units that contain more than 15 µg ethinyl estradiol, the present invention achieves contraceptive reliability at lower amounts. Hence, preferably, the daily hormone units for use during the estrogenic phase contain the synthetic estrogen in an amount equivalent to 5-15 µg ethinyl estradiol.

The application of relatively low levels of estrogen offers the advantage that it minimises the risk of estrogenic side effects. Examples of side effects associated with the administration of estrogens are nausea, vomiting, breast tension, headache, mood disturbances, fluid retention, bloating, liver function disturbances, cholelithiasis, cholestatic icterus, pancreatitis, thromboembolism.

The daily hormone units for use during the progestogenic phase preferably contain the biogenic estrogen in an amount equivalent to 0.5-5 mg 17beta-estradiol. The invention makes it possible to apply relatively low levels of biogenic estrogen. Thus in another preferred embodiment the daily hormone units for use during the progestogenic phase contain the biogenic estrogen in an amount equivalent to 1-3 mg 17beta-estradiol.

The daily hormone units for use during the progestogenic phase may suitably contain the progestogen in an amount equivalent to 30-750 µg levonorgestrel. More preferably the maximum amount of progestogen in the daily unit is less than the equivalent of 250 µg levonorgestrel. The minimum amount of progestogen preferably exceeds the equivalent of 40 µg levonorgestrel. Most preferably the amount of progestogen in the daily unit is equivalent to 75-150 µg levonorgestrel.

In order to determine for a specific biogenic estrogen or synthetic estrogen the amounts that are equivalent to a cited amount of ethinyl estradiol or 17beta-estradiol, the method described by Allen and Doisy may suitably be used (Allen A., Doisy E.A., "An ovarian hormone. Preliminary report on its localization, extraction and partial purification, and action in test animals. JAMA (1923), 81, 819-821). Similarly, in order to determine fora given progestogen the amount equivalent to cited amount of levonorgestrel the method originally described by McPhail can be used (Mc Phail M.K. "The assay of progestin" J Physiol (1934), 83, 145-156). A more recent description of this method can be found in an article written by Overbeek G.A., de Visser J. "A new substance with progestational activity", Acta Endocrinol (1956), 22, 318-329. It is noted that the aforementioned methods will provide useful indications about the anticipated estrogen potency or progestogen potency of a particular hormone. However, to accurately determine the equivalent amounts that are referred to above, it is advisable to additionally conduct in vivo studies in human females.

For guidance the table below provides the conversion factors for a number of progestogens that may suitably be used in the method of the invention. These conversion factors may be used to calculate, for each progestogen mentioned in the table, an estimate of the amount of said progestogen which is equivalent to a given amount of levonorgestrel.

| | Conversion factor | equivalent to 30 µg levonorgestrel | equivalent to 750 µg levonorgestrel |
|---|---|---|---|
| levonorgestrel | 1 | 30 µg | 750 µg |
| norethisterone | 7 | 210 µg | 5.25 mg |
| norgestimate | 1.7 | 51 µg | 1.275 mg |
| drospirenone | 20 | 600 µg | 15 mg |
| dydrogesterone | 133 | 4 mg | 100 mg |

The synthetic estrogen present in the kit according to the invention is preferably selected from the group consisting of: ethinyl estradiol, mestranol, quinestranol, precursors capable of liberating such an estrogen when used in the present contraceptive method and mixtures thereof. Most preferably the synthetic estrogen is ethinyl estradiol or a precursor capable of liberating ethinyl estradiol. The biogenic estrogen is preferably selected from the group consisting of: estradiol, estrone, estran, estriol, estetrol, conjugated equine estrogens, precursors capable of liberating such an estrogen when used in the present method and mixtures thereof. Most preferably the biogenic estrogen is estradiol or a precursor capable of liberating estradiol. Here the term estradiol encompasses both 17alpha-estradiol and 17beta-estradiol. Most preferably the biogenic estrogen is 17beta-estradol or a precursor thereof.

The progestogen contained in the kit of the invention is preferably selected from the group consisting of levonorgestrel, norgestimate, norethisterone, dydrogesterone, drospirenone, 3-beta-hydroxydesogestrel, 3-keto desogestrel (=etonogestrel), 17-deacetyl norgestimate, 19-norprogesterone, acetoxypregnenolone, allylestrenol, anagestone, chlormadinone, cyproterone, demegestone, desogestrel, dienogest, dihydrogesterone, dimethisterone, ethisterone, ethynodiol diacetate, flurogestone acetate, gastrinon, gestodene, gestrinone, hydroxymethylprogesterone, hydroxyprogesterone, lynestrenol (=lynoestrenol), medrogestone, medroxyprogesterone, megestrol, melengestrol, nomegestrol, norethindrone (=norethisterone), norethynodrel, norgestrel (includes d-norgestrel and dl norgestrel), norgestrienone, normethisterone, progesterone, quingestanol, (17alpha)-17-hydroxy-11-methylene-19-norpregna-4,15-diene-20-yn-3-one, tibolone, algestone acetophenide, nestorone, promegestone, 17-hydroxyprogesterone esters, 19-nor-17hydroxyprogesterone, 17alpha-ethinyl-testosterone, 17alpha-ethinyl-19-nor-testosterone, d-17beta-acetoxy-13beta-ethyl-17alpha-ethinyl-gon-4-en-3-one oxime and precursors of these compounds. Preferably the progestogen used in the progestogenic phase is selected from the group consisting of levonorgestrel, norgestimate, norethisterone, drospirenone, dydrogesterone and their precursors

Specific examples of progestogen precursor which may be employed in accordance with the present invention include: anagestone acetate, chlormadinone acetate, cyproterone acetate, gestodene acetate, hydroxymethylprogesterone acetate, hydroxyprogesterone acetate, hydroxyprogesterone hexanoate, hydroxyprogesterone caproate, hydroxyprogesterone enanthate, medroxyprogesterone acetate, megestrol acetate, melengestrol acetate, nomegestrol acetate, norethindrone acetate, norethisterone acetate, norethisterone enanthate, quingestanol acetate, (17alpha)-17-hydroxy-11-methylene-19-norpregna-4,15-diene-20-yn-3-one, tibolone, algestone acetophenide, nestorone, promegestone, 17-hydroxyprogesterone esters, 19-nor-17hydroxyprogesterone esters, 17alpha-ethinyl-testosterone.

Best results are obtained with the kit according to the invention when the synthetic estrogen is ethinyl estradiol or a precursor thereof, the biogenic estrogen is estradiol or a precursor thereof and the progestogen is selected from the group consisting of levonorgestrel, norgestimate, norethisterone, drospirenone, dydrogesterone and their precursors. Thus in a preferred embodiment the hormone units for use during the estrogenic phase contain ethinyl estradiol or a combination of ethinyl estradiol and estradiol and/or precursors thereof in a therapeutically effective amount to inhibit ovulation and the hormone units for use during the progestogenic phase contain a therapeutically effective amount of a combination of estradiol and/or a precursor thereof and a progestogen selected from the group consisting of levonorgestrel, norgestimate, norethisterone, drospirenone, dydrogesterone and their precursors, to inhibit ovulation and to transform the endometrium from a proliferative into a secretory state. Of the latter progestogens levonorgestrel and norgestimate are particularly preferred.

Throughout this document by precursors of an active ingredient are meant components capable of liberating the active ingredient when used in the present contraceptive method, particularly after administration e.g. as a result of metabolic conversion of the precursor substance. Particularly useful precursors of the hormones present in the kit according to the invention are substances that differ from these hormones in that the hydrogen in at least one of the hydroxyl groups in the hormone-molecule has been substituted by -CO-R, wherein R is a hydrocarbon radical comprising from 1-25 carbons.

It is to be understood that the present invention not only encompasses the use of estrogens and progestogens specifically mentioned in this application, but also metabolites of these hormones that display comparable functionality. In this context it is noted that, for instance, levonorgestrel is a metabolite of norgestimate and that estriol is a metabolite of 17beta-estradiol. Both these progestogens and estrogens have found application in contraceptive formulations and/or preparations for hormone replacement therapy. The plurality of hormone units contained by the present kit can suitably consist of 1-18 daily units for use in the estrogenic phase and 10-27 daily units for use in the progestogenic phase. More preferably the present kit consists of 10-16 daily units for use in the estrogenic phase and 12-18 daily units for use in the progestogenic phase. Most preferably the kit consists of 13-15 daily units for use in the estrogenic phase and 13-15 units for use in the progestogenic phase. The hormone units are preferably for oral administration and arranged in a fixed sequence corresponding to the intended order of administration in 2 phases. Preferably, the hormone units to be used in either the estrogenic or progestogenic phase are easily distinguishable, e.g. because they are different in colour and/or shape. Data indications may be provided on the packaging. The packaging may be a tube or box or a strip. The box may be circular, square, or otherwise shaped with the tablets being accommodated separately therein for ease of administration. Date indications may appear adjacent to each tablet corresponding with the days on which each tablet is to be taken. Some indication of the sequence in which the tablets are to be taken preferably appears on the packaging regardless of its form.

Generally speaking, the hormone units in the present kit are prepared according to conventionally known procedures in accordance with the method of administration. Thus, the active ingredients are prepared according to known methods in a pharmaceutically acceptable form for administration. These ingredients, in their required quantities are combined with the appropriate pharmaceutical carriers such as additives, vehicles and/or flavour ameliorating substances. These substances may be referred to as diluents, binders and lubricants. Gums, starches and sugars are also common terms. Typical of these types of substances or excipients are pharmaceutical grades of mannitol, lactose starch, magnesium stearate, sodium saccharin, talcum, cellulose, glucose, sucrose, magnesium carbonate and the like. The active ingredient(s) may comprise from about 0.01 % by weight to about 99.99% by weight of the total formulation and the remainder comprises the pharmaceutically acceptable carrier. The percentage of active ingredient(s) may vary according to the delivery system or method of administration and is chosen in accordance with conventional methods known in the art. Thus, the active ingredients are compounded with the chosen carrier and in for example the case of a tablet form, placed in a tablet moulding apparatus to form the tablets which are subsequently packaged in accordance with the chosen regimen.

Another aspect of the present invention relates to the use of synthetic estrogen, biogenic estrogen and progestogen in the manufacture of a kit containing hormone units for use in a contraceptive method that consists of two alternating consecutive phases - an estrogenic and a progestogenic phase - said method comprising administering to a female of childbearing capability
a) during the estrogenic phase one or more hormone units to provide a therapeutically effective amount of synthetic estrogen or a combination of synthetic estrogen and biogenic estrogen to inhibit ovulation and
b) during the progestogenic phase one or more hormone units to provide a combination of biogenic estrogen and progestogen in a therapeutically effective amount to inhibit ovulation and to transform the endometrium from a proliferative into a secretory state,
wherein the progestogenic phase encompasses a period of at least 10 days and the two consecutive phases together encompass a period of 20-35 days.

In the following examples, specific embodiments of the present invention are set forth. These are meant to be illustrative of the invention and are not meant to limit it in any way.

### EXAMPLES

### Example 1

Women participating in the study described in these examples are all selected on the basis that they are users of'high' dose monophasic ethinyl estradiol-containing combined oral contraceptives. The reason for applying this selection criterion is that there is a dose-relationship between follicular development and ethinyl estradiol whether given alone or in combination with a progestogen, i.e.less follicular development in the presence of the higher dose. Participation of the females in the study starts at a moment of (almost) complete suppression of follicular development. This condition is not met if a female has her first day of bleeding in a natural cycle, after a tablet-free interval, or immediately after a low-dose combined oral contraceptive.

A clinical study in contraception is conducted in 16 healthy young women who previously used a monophasic combined oral contraceptive pill with at least 30 microgram of ethinyl estradiol. Eight women are administered ethinyl estradiol at a dose of 10 microgram/day during days 1-14 of the cycle. Another eight women are administered 15 microgram/day of ethinyl estradiol during days 1-14 of the cycle. From days 15-28 of the cycle, 10 mg of dydrogesterone combined with 1 mg 17beta-estradiol is administered to the women of both (ethinyl estradiol) dose groups. The participants are followed by vaginal ultrasonography and blood sampling for endogenous hormones to assess ovarian function (ovulation inhibition). Vaginal bleeding pattern and feeling of well being are scored by the participants.

Results show that the ovarian function is suppressed to the extent of ovulation inhibition. The follicular development is more profoundly suppressed in the 15 microgram ethinyl estradiol group than in the 10 microgram group. There is no intermenstrual bleeding in either group and participants reported an improved feeling of well being.

### Example 2

Another study is conducted with 8 healthy young women who were selected on the basis of the same criteria as mentioned in Example 1. During days 1-14 of the cycle they are administered 10 microgram/day of ethinyl estradiol followed by a combination of 1 mg 17beta-estradiol with 1 mg norethisterone acetate during days 15-28. Again the participants are followed by vaginal ultrasonography and blood sampling for endogenous hormones to assess ovarian function (ovulation inhibition). Vaginal bleeding pattern and feeling of well being are scored by the participants.

Results show that ovarian function was suppressed to the extent of ovulation inhibition. There is no intermenstrual bleeding and participants reported an improved feeling of well being.

### Example 3

Another clinical study is conducted with 8 healthy young women who were selected on the basis of the same criteria as mentioned in Example 1. During days 1-14 of the cycle they are administered 10 microgram/day of ethinyl estradiol, followed by a combination of 1 mg 17beta-estradiol with 100 microgram levonorgestrel during days 15-28. Again the participants are followed by vaginal ultrasonography and blood sampling for endogenous hormones to assess ovarian function (ovulation inhibition). Vaginal bleeding pattern and feeling of well being are scored by the participants.

Results show that ovarian function was suppressed to the extent of ovulation inhibition. There is no intermenstrual bleeding and participants reported an improved feeling of well being.

### Example 4

Yet another clinical study is conducted with 8 healthy young women who were selected on the basis of the same criteria as mentioned in Example 1. During days 1-14 of the cycle they are administered 5 microgram/day of ethinyl estradiol in combination with 1 mg 17beta-estradiol, followed by a combination of 1 mg 17beta-estradiol with 100 microgram levonorgestrel during days 15-28. Again the participants are followed by vaginal ultrasonography and blood sampling for endogenous hormones to assess ovarian function (ovulation inhibition). Vaginal bleeding pattern and feeling of well being are scored by the participants.

Results show that ovarian function was suppressed to the extent of ovulation inhibition. There is no intermenstrual bleeding and participants reported an improved feeling of well being.

## Claims

1. A kit containing a plurality of daily hormone units for use in a contraceptive method, the plurality of daily hormone units consisting of:
a) one or more daily hormone units, for use during an estrogenic phase, containing a synthetic estrogen or a combination of synthetic estrogen and biogenic estrogen in an amount equivalent to 3-40 µg ethinyl estradiol and
b) at least 10 daily hormone units, for use during a progestogenic phase, containing biogenic estrogen in an amount equivalent to 0.5-5 mg 17beta-estradiol and progestogen in an amount equivalent to 30 - 750 µg levonorgestrel.

2. A kit containing a plurality of hormone units for use in a contraceptive method that consists of two alternating consecutive phases - an estrogenic and a progestogenic phase - said method comprising administering to a female of childbearing capability
a) during the estrogenic phase one or more hormone units to provide a therapeutically effective amount of synthetic estrogen or a combination of synthetic estrogen and biogenic estrogen to inhibit ovulation and
b) during the progestogenic phase one or more hormone units to provide a combination of biogenic estrogen and progestogen in a therapeutically effective amount to inhibit ovulation and to transform the endometrium from a proliferative into a secretory state,
wherein the progestogenic phase encompasses a period of at least 10 days and the two consecutive phases together encompass a period of 20-35 days.

3. A kit according to claim 1 or 2, wherein the plurality of hormone units consists of 20 to 35 daily hormone units, preferably 28 daily hormone units

4. A kit according to any one of claims 1-3, wherein the units for use during the estrogenic phase also contain a biogenic estrogen.

5. A kit according to any one of claims 1-4, wherein the units for use during the progestogenic phase do not contain a synthetic estrogen.

6. A kit according to any one of claims 2-5, wherein the daily hormone units for use during the estrogenic phase contain the synthetic estrogen in an amount equivalent to 3-40 µg, preferably 5-15 µg ethinyl estradiol.

7. A kit according to any one of claims 2-6, wherein the daily hormone units for use during the progestogenic phase contain the biogenic estrogen in an amount equivalent to 0.5-5 mg, preferably 1-3 mg 17beta-estradiol.

8. A kit according to any one of claims 2-7, wherein the daily hormone units for use during the progestogenic phase contain the progestogen in an amount equivalent to 30-750 µg, preferably -75-150 µg levonorgestrel.

9. A kit according to any one of claims 1-8, wherein the synthetic estrogen is selected from the group consisting of: ethinyl estradiol, mestranol, quinestranol, precursors capable of liberating such an estrogen when used in the present method and mixtures thereof.

10. A kit according to any one of claims 1-9, wherein the biogenic estrogen is selected from the group consisting of: estradiol, estrone, estran, estriol, estetrol, conjugated equine estrogens, precursors capable of liberating such an estrogen when used in the present method and mixtures thereof.

11. A kit according to any one of claims 1-10, wherein the progestogen is selected from the group consisting of levonorgestrel, norgestimate, norethisterone, dydrogesterone, drospirenone, 3-beta-hydroxydesogestrel, etonogestrel, 17-deacetyl norgestimate, 19-norprogesterone, acetoxypregnenolone, allylestrenol, anagestone, chlormadinone, cyproterone, demegestone, desogestrel, dienogest, dihydrogesterone, dimethisterone, ethisterone, ethynodiol diacetate, flurogestone acetate, gastrinon, gestodene, gestrinone, hydroxymethylprogesterone, hydroxyprogesterone, lynestrenol, medrogestone, medroxyprogesterone, megestrol, melengestrol, nomegestrol, norethindrone, norethynodrel, norgestrel, norgestrienone, normethisterone, progesterone, quingestanol, (17alpha)-17-hydroxy-11-methylene-19-norpregna-4,15-diene-20-yn-3-one, tibolone, algestone acetophenide, nestorone, promegestone, 17-hydroxyprogesterone esters, 19-nor-17hydroxyprogesterone, 17alpha-ethinyl-testosterone, 17alpha-ethinyl-19-nor-testosterone, d-17beta-acetoxy-13beta-ethyl-17alpha-ethinyl-gon-4-en-3-one oxime and precursors of these compounds.

12. A kit according to any one of claims 1-11, wherein the hormone units for use during the estrogenic phase contain ethinyl estradiol or a combination of ethinyl estradiol and estradiol and/or precursors thereof in a therapeutically effective amount to inhibit ovulation and the hormone units for use during the progestogenic phase contain a combination of estradiol and/or a precursor thereof and a progestogen selected from the group consisting of levonorgestrel, norgestimate, norethisterone, drospirenone, dydrogesterone and their precursors, in a therapeutically effective amount to inhibit ovulation and to transform the endometrium from a proliferative into a secretory state.

13. A kit according to any one of claims 1-12, wherein the plurality of daily hormone units consists of 1-18 units for use in the estrogenic phase and 10-27 units for use in the progestogenic phase.

14. Use of synthetic estrogen, biogenic estrogen and progestogen in the manufacture of a kit containing hormone units for use in a contraceptive method that consists of two alternating consecutive phases - an estrogenic and a progestogenic phase - said method comprising administering to a female of childbearing capability
a) during the estrogenic phase one or more hormone units to provide a therapeutically effective amount of synthetic estrogen or a combination of synthetic estrogen and biogenic estrogen to inhibit ovulation and
b) during the progestogenic phase one or more hormone units to provide a combination of biogenic estrogen and progestogen in a therapeutically effective amount to inhibit ovulation and to transform the endometrium from a proliferative into a secretory state,
wherein the progestogenic phase encompasses a period of at least 10 days and the two consecutive phases together encompass a period of 20-35 days.

15. Use according to claim 14, wherein the units for use during the estrogenic phase also contain a biogenic estrogen.

16. Use according to claim 14 or 15, wherein the units for use during the progestogenic phase do not contain a synthetic estrogen.

17. Use according to any one of claims 14-16, wherein the daily hormone units for use during the estrogenic phase contain the synthetic estrogen in an amount equivalent to 3-40 µg, preferably 5-15 µg ethinyl estradiol.

18. Use according to any one of claims 14-17, wherein the daily hormone units for use during the progestogenic phase contain the biogenic estrogen in an amount equivalent to 0.5-5 mg, preferably 1-3 mg 17beta-estradiol.

19. Use according to any one of claims 14-18, wherein the daily hormone units for use during the progestogenic phase contain the progestogen in an amount equivalent to 30-750 µg, preferably 75-150 µg levonorgestrel.

20. Use according to any one of claims 14-19, wherein the hormone units for use during the estrogenic phase contain ethinyl estradiol or a combination of ethinyl estradiol and estradiol and/or precursors thereof in a therapeutically effective amount to inhibit ovulation and the hormone units for use during the progestogenic phase contain a therapeutically effective amount of a combination of estradiol and/or a precursor thereof and a progestogen selected from the group consisting of levonorgestrel, norgestimate, norethisterone, drospirenone, dydrogesterone and their precursors, to inhibit ovulation and to transform the endometrium from a proliferative into a secretory state.

21. Use according to any one of claims 14-20, wherein the plurality of daily hormone units consists of 1-18 units for use in the estrogenic phase and 10-27 units for use in the progestogenic phase.

## Revendications

1. Conditionnement contenant une pluralité d'unités journalières d'hormone pour une utilisation dans une méthode contraceptive, la pluralité des unités d'hormone journalière étant composée de :
a) une ou plusieurs unités journalières d'hormone, pour utilisation pendant une phase oestrogénique, contenant un oestrogène synthétique ou une combinaison d'oestrogène synthétique et d'oestrogène biogénique dans une quantité équivalente à 3 à 40 µg d'éthinyl estradiol et
b) au moins 10 unités journalières d'hormone, pour utilisation pendant une phase progestogénique, contenant un oestrogène biogénique dans une quantité équivalente à 0,5 à 5 mg de 17bêta estradiol et un progestogène dans une quantité équivalente à 30 à 750 µg de lévonorgestrel.

2. Conditionnement contenant une pluralité d'unités d'hormone pour utilisation dans un procédé contraceptif qui consiste en deux phases consécutives alternées - une phase oestrogénique et une phase progestogénique - ledit procédé étant constitué par l'administration à une femme ayant la capacité à porter un enfant
a) pendant la phase oestrogénique une ou plusieurs unités d'hormone pour fournir une quantité thérapeutiquement efficace d'oestrogène synthétique ou une combinaison d'oestrogène synthétique et d'oestrogène biogénique pour inhiber l'ovulation et
b) pendant la phase progestogénique une ou plusieurs unités d'hormone pour fournir une combinaison d'oestrogène biogénique et de progestogène dans une quantité thérapeutiquement efficace pour inhiber l'ovulation et pour transformer l'endométre d'un état prolifératif à un état sécrétoire,
dans lequel la phase progestogénique comprend une période d'au moins 10 jours et les deux phases consécutives ensemble comprennent une période de 20 à 35 jours.

3. Conditionnement selon la revendication 1 ou la revendication 2, dans lequel la pluralité d'unités d'hormone est composée de 20 à 35 unités journalières d'hormone, de préférence 28 unités journalières d'hormone.

4. Conditionnement selon l'une quelconque des revendications 1 à 3, dans lequel les unités pour utilisation pendant la phase oestrogénique contiennent aussi un oestrogène biogénique.

5. Conditionnement selon l'une quelconque des revendications 1 à 4, dans lequel les unités pour utilisation pendant la phase progestogénique ne contiennent pas d'oestrogène synthétique.

6. Conditionnement selon l'une quelconque des revendications 2 à 5, dans lequel les unités journalières d'hormone pour utilisation pendant la phase oestrogénique contiennent l'oestrogène synthétique dans une quantité équivalente à 3 à 40 µg, de préférence 5 à 15 µg d'éthinyl estradiol.

7. Conditionnement selon l'une quelconque des revendications 2 à 6, dans lequel les unités journalières d'hormone pour utilisation pendant la phase progestrogénique contiennent l'oestrogène biogénique dans une quantité équivalente à 0,5 à 5 mg, de préférence 1 à 3 mg de 17 bêta- estradiol.

8. Conditionnement selon l'une quelconque des revendications 2 à 7, dans lequel les unités journalières d'hormone pour utilisation pendant la phase progestogénique contiennent le progestogène dans une quantité équivalente à 30 à 750 µg, de préférence 75 à 150 µg de lévonorgestrel.

9. Conditionnement selon l'une quelconque des revendications 1 à 8, dans lequel l'oestrogène synthétique est choisi parmi le groupe composé de : l'éthinyl estradiol, le mestranol, le quinestranol, les précurseurs capables de libérer un tel oestrogène lorsqu'ils sont utilisés dans le présent procédé ainsi que leurs mélanges.

10. Conditionnement selon l'une quelconque des revendications 1 à 9, dans lequel l'oestrogène biogénique est choisi parmi le groupe composé de : l'estradiol, l'estrone, l'estran, l'estriol, l'estétrol, les oestrogènes conjugués équins, les précurseurs capables de libérer un tel oestrogène lorsqu'ils sont utilisés dans le présent procédé et leurs mélanges.

11. Conditionnement selon l'une quelconque des revendications 1 à 10, dans lequel le progestogène est choisi parmi le groupe composé du lévonorgestrel, du norgestimate, de la noréthistérone, de la dydrogestérone, de la drospirénone, du 3-bêta-hydroxydésogestrel, de l'étonogestrel, du 17-déacétyl norgestimate, de la 19-norprogestérone, de l'acétoxyprégnénolone, de l'allylestrénol, de l'anagestone, de la chlormadinone, de la cyprotérone, de la démégestone, du désogestrel, du diénogest, de la dihydrogestérone, de la diméthistérone, de l'ét'istérone, du diacétate d'éthynodiol, de l'acétate de flurogestone, du gastrinon, du gestodène, de la gestrinone, de l'hydroxyméthylprogestérone, de l'hydroxyprogestérone, du lynestrénol, de la médrogestone, de la médroxyprogestérone, du mégestrol, du mélengestrol, du nomégestrol, de la noréthindrone, du noréthynodrel, du norgestrel, de la norgestriénone, de la norméthistérone, de la progestérone, du quingestanol, de la (17alpha)-17-hydroxy-11-méthylène-19-norprégna-4,15-diène-20-yn-3-one, de la tibolone, de l'acétophénide d'algestone, de la nestorone, de la promégestone, des esters de 17-hydroxyprogestérone, de la 19-nor-17hydroxyprogestérone, de la 17alpha-éthinyl-testostérone, de la 17alpha-éthinyl-19-nor-testostérone, du d-17bêta-acétoxy-13bêta-éthyl-17alpha-éthinyl-gon-4-en-3-one et les précurseurs de ces composés.

12. Conditionnement selon l'une quelconque des revendications 1 à 11, dans lequel les unités d'hormone pour utilisation pendant la phase oestrogénique contiennent de l'éthinyl estradiol ou une combinaison d'éthinyl estradiol et d'estradiol et/ou leurs précurseurs dans une quantité thérapeutiquement efficace pour inhiber l'ovulation et les unités d'hormone pour utilisation pendant la phase progestogénique contiennent une combinaison d'estradiol et/ou son précurseur et un progestogène choisi parmi le groupe composé du lévonorgestrel, du norgestimate, de la noréthistérone, de la drospirénone, de la dydrogestérone et leurs précurseurs, dans une quantité thérapeutiquement efficace pour inhiber l'ovulation et pour transformer l'endomètre d'un état prolifératif à un état sécrétoire.

13. Conditionnement selon l'une quelconque des revendications 1 à 12, dans lequel la pluralité des unités journalières d'hormone est composée de 1 à 18 unités pour utilisation dans la phase oestrogénique et 10 à 27 unités pour utilisation dans la phase progestogénique.

14. Utilisation d'un oestrogène synthétique, d'un oestrogène biogénique et d'un progestogène dans la fabrication d'un conditionnement contenant des unités d'hormone pour utilisation dans un procédé contraceptif qui est composé de deux phases alternées consécutives - une phase oestrogénique et une phase progestogénique - ledit procédé comportant l'administration à une femmè ayant la capacité à porter un enfant.
a) pendant la phase oestrogénique une ou plusieurs unités d'hormone pour fournir un quantité thérapeutiquement efficace d'oestrogène synthétique ou une combinaison d'oestrogène synthétique et d'oestrogène biogénique pour inhiber l'ovulation et
b) pendant la phase progestogénique une ou plusieurs unités d'hormone pour fournir une combinaison d'oestrogène biogénique et de progestogène dans une quantité thérapeutiquement efficace pour inhiber l'ovulation et pour transformer l'endomètre d'un état prolifératif en un état sécrétoire,
dans lequel la phase progestogénique comprend une période d'au moins 10 jours et les deux phases consécutives ensemble comprennent une période de 20 à 35 jours.

15. Utilisation selon la revendication 14, dans laquelle les unités pour utilisation pendant la phase oestrogénique contiennent aussi un oestrogène biogénique.

16. Utilisation selon la revendication 14 ou la revendication 15, dans laquelle les unités pour utilisation pendant la phase progestogénique ne contiennent pas un oestrogène synthétique.

17. Utilisation selon l'une quelconque des revendications 14 à 16, dans laquelle les unités journalières d'hormone pour utilisation pendant la phase oestrogénique contiennent l'oestrogène synthétique dans une quantité équivalente à 3 à 40 µg, de préférence 5 à 15 µg d'éthinyl estradiol.

18. Utilisation selon l'une quelconque des revendications 14 à 17, dans laquelle les unités journalières d'hormone pour utilisation pendant la phase progestogénique contiennent l'oestrogène biogénique dans une quantité équivalente à 0,5 à 5 mg, de préférence 1 à 3 mg de 17bêta-estradiol.

19. Utilisation selon l'une quelconque des revendications 14 à 18, dans laquelle les unités journalières d'hormone pour utilisation pendant la phase progeatogénique contiennent le progestogène dans une quantité équivalente à 30 à 750 µg, de préférence 75 à 150 µg de lévonorgestrel.

20. Utilisation selon l'une quelconque des revendications 14 à 19, dans laquelle les unités d'hormone pour utilisation pendant la phase oestrogénique contiennent de l'éthinyl estradiol ou une combinaison d'éthinyl estradiol et d'estradiol et/ou leurs précurseurs dans une quantité thérapeutiquement efficace pour inhiber l'ovulation et les unités d'hormone pour utilisation pendant la phase progestogénique contiennent une quantité thérapeutiquement efficace d'une combinaison d'estradiol et/ou son précurseur et un progestogène choisi parmi le groupe composé de lévonorgestrel, de norgestimate, de noréthistérone, de drospirénone, de dydrogestérone et de leur précurseurs, pour inhiber l'ovulation et pour transformer l'endomètre d'un état prolifératif en un état sécrétoire.

21. Utilisation selon l'une quelconque des revendications 14 à 20, dans laquelle la pluralité d'unités journalières d'hormone est composée de 1 à 18 unités pour utilisation dans la phase oestrogénique et de 10 à 27 unités pour utilisation dans la phase progestogénique.

## Patentansprüche

1. Satz mit einer Vielzahl täglicher Hormoneinheiten zur Verwendung in einer Verhütungsmethode, wobei die Vielzahl der täglichen Hormoneinheiten aus folgenden Bestandteilen besteht:
a) eine oder mehrere tägliche Hormoneinheiten zur Verwendung während einer östrogenen Phase, welche synthetisches Östrogen oder eine Kombination aus synthetischem Östrogen und biogenem Östrogen in einer Höhe enthalten, die 3-40 µg Ethinylöstradiol gleichwertig ist, und
b) mindestens 10 tägliche Hormoneinheiten zur Verwendung während einer progestogenen Phase, welche biogenes Östrogen in einer Höhe, die 0,5-5 mg 17β-östradiol gleichwertig ist, und Progestogen in einer Höhe, die 30-750 µg Levonorgestrel gleichwertig ist, enthalten.

2. Satz mit einer Vielzahl von Hormoneinheiten zur Verwendung in einer Verhütungsmethode, die aus zwei abwechselnd aufeinander folgenden Phasen besteht - einer östrogenen und einer progestogenen Phase -, wobei diese Methode die Verabreichung folgender Hormone an eine gebärfähige Frau umfasst:
a) während der östrogenen Phase eine oder mehrere Hormoneinheiten, welche eine therapeutisch wirksame Menge von synthetischem Östrogen oder eine Kombination aus synthetischem Östrogen und biogenem Östrogen enthalten, um den Eisprung zu verhindern, und
b) während der progestogenen Phase eine oder mehrere Hormoneinheiten, die eine Kombination aus biogenem Östrogen und Progestogen in einer therapeutisch wirksamen Höhe enthalten, um den Eisprung zu verhindern und die Gebärmutterschleimhaut von einem fruchtbaren in einen sekretorischen Zustand zu transformieren.
wobei die progestogene Phase eine Zeitspanne von mindestens 10 Tagen umfasst, und die zwei aufeinander folgenden Phasen zusammen eine Zeitspanne von 20-35 Tagen umfassen.

3. Satz nach Anspruch 1 oder 2, wobei die Vielzahl der Hormoneinheiten aus 20 bis 35 täglichen Hormoneinheiten besteht, vorzugsweise aus 28 täglichen Hormoneinheiten.

4. Satz nach einem der Ansprüche 1-3, wobei die Einheiten zur Verwendung während der östrogenen Phase auch ein biogenes Östrogen enthalten.

5. Satz nach einem der Ansprüche 1-4, wobei die Einheiten zur Verwendung während der progestogenen Phase kein synthetisches Östrogen enthalten.

6. Satz nach einem der Ansprüche 2-5, wobei die täglichen Hormoneinheiten zur Verwendung während der östrogenen Phase das synthetische Östrogen in einer Höhe enthalten, die 3-40 µg, vorzugsweise 5-15 µg, Ethinylöstradiol gleichwertig ist.

7. Satz nach einem der Ansprüche 2-6, wobei die täglichen Hormoneinheiten zur Verwendung während der progestogenen Phase das biogene Östrogen in einer Höhe enthalten, die 0,5-5 mg, vorzugsweise 1-3 mg, 17β-Östradiol gleichwertig ist.

8. Satz nach einem der Ansprüche 2-7, wobei die täglichen Hormoneinheiten zur Verwendung während der progestogenen Phase das Progestogen in einer Höhe enthalten, die 30-750 µg, vorzugsweise 75-150 µg, Levonorgestrel gleichwertig ist.

9. Satz nach einem der Ansprüche 1-8, wobei das synthetische Östrogen aus der folgenden Gruppe gewählt ist: Ethinylöstradiol, Möstranol, Quinöetranol, Vorläufer, die in der Lage sind, ein solches Östrogen freizusetzen, wenn sie in der vorliegenden Methode und deren Mischungen verwendet sind.

10. Satz nach einem der Ansprüche 1-9, wobei das biogene Östrogen aus der folgenden Gruppe gewählt ist: Östradiol, Östron, Östran, Östriol, Östetrol, konjugierte Pferdeöstrogene, Vorläufer, die in der Lage sind, ein solches östrogen freizusetzen, wenn sie in der vorliegenden Methode verwendet werden und deren Mischungen.

11. Satz nach einem der Ansprüche 1-10, wobei das Progestogen ausgewählt ist aus der Gruppe von Levonorgestrel, Norgestimat, Norethisteron, Dydrogesteron, Drospirenon, 3β-Hydroxydesogestrel, Etonogestrel, 17-Deacetylnorgestimat, 19-Norprogesteron, Acetoxypregnenolon, Allylestrenol, Anageston, Chlormadinon, Cyproteron, Demegeston, Desogestrel, Dienogest, Dihydrogesteron, Dimethisteron, Ethisteron, Ethynodioldiacetat, Flurogestonacetat, Gastrinon, Gestoden, Gestrinon, Hydroxymethylprogesteron, Hydroxyprogesteron, Lynöstrenol, Medrogeston, Medroxyprogesteron, Megestrol, Melengestrol, Nomegestrol, Norethindron, Norethynodrel, Norgestrel, Norgestrienon, Normethisteron, Progesteron, Quingestanol, (17α) -17-Hydroxy-11-methylen-19-norpregna-4,15-dien-20-yn-3-on, Tibolon, Algestonacetophenid, Nestoron, Promegeston, 17-Hydroxyprogesteronester, 19-Nor-17-hydroxyprogesteron, 17α-Ethinyltestosteron, 17α-Ethinyl-19-nor-testosteron, d-17β-Acetoxy-13β-ethyl-17α-ethinyl-gon-4-en-3-on-oxim und Vorläufer dieser Verbindungen.

12. Satz nach einem der Ansprüche 1-11, wobei die Hormoneinheiten zur Verwendung während der östrogenen Phase Ethinylöstradiol und Östradiol und/oder Vorläufer derselben in einer therapeutisch wirksamen Höhe enthalten, um den Eisprung zu verhindern, und die Hormoneinheiten für die Verwendung während der progestogenen Phase eine Kombination aus Östradiol und/oder einen Vorläufer desselben und ein Progestogen aus der Gruppe von Levonorgestrel, Norgestimat, Norethisteron, Drospirenon, Dydrogesteron und deren Vorläufer in einer therapeutisch wirksamen Höhe enthalten, um den Eisprung zu verhindern und die Gebärmutterschleimhaut von einem fruchtbaren in einen sekretorischen Zustand zu transformieren.

13. Satz nach einem der Ansprüche 1-12, wobei die Vielzahl der täglichen Hormoneinheiten aus 1-18 Einheiten zur Verwendung in der östrogenen Phase und 10-27 Einheiten zur Verwendung in der progestogenen Phase besteht.

14. Verwendung von synthetischem Östrogen, biogenem Östrogen und Progestogen bei der Herstellung eines Satzes Hormoneinheiten zur Verwendung in einer verhütungsmethode, die aus zwei abwechselnd aufeinander folgenden Phasen besteht - einer östrogenen und einer progestogenen Phase -, wobei diese Methode die Verabreichung folgender Hormone an eine gebärfähige Frau umfasst:
a) während der östrogenen Phase eine oder mehrere Hormoneinheiten, welche eine therapeutisch wirksame Menge von synthetischem Östrogen oder eine Kombination aus synthetischem Östrogen und biogenem östrogen enthalten, um den Eisprung zu verhindern, und
b) während der progestogenen Phase eine oder mehrere Hormoneinheiten, die eine Kombination aus biogenem Östrogen und Progestogen in einer therapeutisch wirksamen Menge enthalten, um den Eisprung zu verhindern und die Gebärmutterschleimhaut von einem fruchtbaren in einen sekretorischen Zustand zu transformieren,
wobei die progestogene Phase eine Zeitspanne von mindestens 10 Tagen umfasst und die zwei aufeinander folgenden Phasen zusammen eine Zeitspanne von 20-35 Tagen umfassen.

15. Verwendung nach Anspruch 14, wobei die Einheiten zur Verwendung während der östrogenen Phase auch ein biogenes Östrogen enthalten.

16. Verwendung nach Anspruch 14 oder 15, wobei die Einheiten zur Verwendung während der progestogenen Phase kein synthetisches Östrogen enthalten.

17. Verwendung nach einem der Ansprüche 14-16, wobei die täglichen Hormoneinheiten zur Verwendung während der östrogenen Phase das synthetische Östrogen in einer Höhe enthalten, die 3-40 µg, vorzugsweise 5-15 µg, Ethinylöstradiol gleichwertig ist.

18. Verwendung nach einem der Ansprüche 14-17, wobei die täglichen Hormoneinheiten zur Verwendung während der progestogenen Phase das biogene Östrogen in einer Höhe enthalten, die 0,5-5 mg, vorzugsweise 1-3 mg, 17β-Östradiol gleichwertig ist.

19. Verwendung nach einem der Ansprüche 14-18, wobei die täglichen Hormoneinheiten zur Verwendung während der progestogenen Phase das Progestogen in einer Höhe enthalten, die 30-750 µg, vorzugsweise 75-150 µg, Levonorgestrel gleichwertig ist.

20. verwendung nach einem der Ansprüche 14-19, wobei die Hormoneinheiten zur Verwendung während der östrogenen Phase Ethinylöstradiol und/oder dessen Vorläufer in einer therapeutisch wirksamen Höhe enthalten, um den Eisprung zu verhindern, und die Hormoneinheiten zur Verwendung während der progestogenen Phase eine Kombination aus Östradiol und/oder dessen Vorläufer und ein Progestogen aus der Gruppe von Levc ogestrel, Norgestimat, Norethisteron, Drospirenon, Dydrogesteron und deren Vorläufer enthalten, um den Eisprung zu verhindern und die Gebärmutterschleimhaut von einem fruchtbaren in einen sekretorischen Zustand zu transformieren.

21. Verwendung nach einem der Ansprüche 14-20, wobei die Vielzahl der täglichen Hormoneinheiten aus 1-18 Einheiten zur Verwendung in der östrogenen Phase und aus 10-27 Einheiten zur Verwendung in der progestogenen Phase besteht.
